**Europäisches Patentamt**

**European Patent Office**

**Office européen des brevets**

(19)

(11) Veröffentlichungsnummer : **0 372 331 B1**

(12)                 **EUROPÄISCHE PATENTSCHRIFT**

(45) Veröffentlichungstag der Patentschrift :
**16.09.92 Patentblatt 92/38**

(51) Int. Cl.⁵ : **A61K 9/107,** A61K 31/685,
A61K 33/26, A61K 33/06,
// (A61K33/26, 31:685, 33:06)

(21) Anmeldenummer : **89121784.6**

(22) Anmeldetag : **25.11.89**

(54) **Stabile Fettemulsion für die parenterale Verabreichung.**

(30) Priorität : **07.12.88 GB 8828517**

(43) Veröffentlichungstag der Anmeldung :
**13.06.90 Patentblatt 90/24**

(45) Bekanntmachung des Hinweises auf die
Patenterteilung :
**16.09.92 Patentblatt 92/38**

(84) Benannte Vertragsstaaten :
**AT BE CH DE FR GB IT LI NL SE**

(56) Entgegenhaltungen :
**EP-A- 0 211 258
CHEMICAL ABSTRACTS, Band 89, 2. Oktober
1978, Seite 523, Zusammenfassung Nr.
117634p, Columbus, Ohio, US; W.H. DAWES et
al.:"The effect of electrolytes on phospholi-
pid-stabilized soybean oil emulsions", & INT.
J. PHARM. 1978, 1(3), 141-50**

(73) Patentinhaber : **LEOPOLD PHARMA
GESELLSCHAFT m.b.H.
Hafnerstrasse 36
A-8055 Graz (AT)**

(72) Erfinder : **Davis, Stanley Stewart, Prof.
19 Cavendish Crescent North
The Park Nottingham (GB)**
Erfinder : **Washington, Clive, Dr.
143 Seaburn Road
Toton Nottingham (GB)**
Erfinder : **Schaupp, Karin Maria, Dr.
Johann Straussgasse 3
A-8010 Graz (AT)**

## Beschreibung

Die nachfolgende Erfindung betrifft eine Fettemulsion für die parenterale Verabreichung, die sich durch Stabilität, auch gegenüber Elektrolyten, auszeichnet.

Seit längerer Zeit sind Bestrebungen im Gange, zur parenteralen Ernährung bei Patienten in kataboler Stoffwechselsituation neben Kohlenhydraten und Aminosäuren Fettemulsionen zur Deckung des Energiebedarfes heranzuziehen. Wissenschaftlich gesehen steht hinter dieser Verwendung auch, daß diese Emulsionen in vivo die natürlichen Blutpartikel, Chylomicronen, nachahmen.

Hierbei war es immer schon das Ziel, solche Fettemulsionen, die bevorzugt Öl-in Wasser-Emulsionen sind, mit den anderen zu verabreichenden Nährstoffen zu einer Gesamtnährlösung zu kombinieren. Diesem Bestreben steht im Wege, daß Fettemulsionen, die in ausreichender Tröpfchenfeinheit und Stabilität aus Öl, z.B. Sojaöl, unter Verwendung von Emulgatoren, insbesondere Lecithinen, wie Eilecithin oder Sojalecithin, herstellbar sind, durch Zusätze polarer Natur, wie saure oder stark basische Aminosäuren oder Elektrolyte, ihre Stabilität einbüßen bzw. zum Brechen neigen.

So werden gemäß DE-B 1,792,294 einer Sojaöl-Emulsion Kohlenhydrate und auch Aminosäuren zugesetzt, wobei aber Arginin ausgeschlossen bleibt, da diese Aminosäure zu Instabilitäten führt. Daneben soll auch der Zusatz von Asparaginsäure und Glutaminsäure vermieden werden. Setzt man diese drei Aminosäuren Fettemulsionen zu, so führen diese trotz eines stabilisierenden Kohlenhydratgehaltes bei einer Ausgangspartikelgröße von 0,1 µm zur Bildung zahlreicher, weitaus größerer Tröpfchen, beispielsweise bis zu 12 µm, die für die intravenöse Applikation abgelehnt werden.

Gemäß DE-C 2,406,621 wird eine Verträglichkeit dieser polaren Aminosäuren für Fettemulsionen dadurch erzielt, daß pro Gramm an als Emulgator eingesetztem Ei-Phospholipid 0,02 bis 3 g einer Fettsäure mit 11 - 20 C-Atomen zugesetzt werden.

Ein noch größeres Problem stellt der Zusatz von Elektrolyten dar, da dieser zu einer zeitabhängigen Instabilität der Fettemulsion führt.

C.D. Black und N.G. Popovich, Drug Intelligence and Clinical Pharmacy 15 (1980) 184 - 193, studierten den Effekt von einwertigen und zweiwertigen Kationen allein oder in Gegenwart von Aminosäuren und/oder Dextrose auf die Stabilität von Fettemulsionen. Hierbei wurde festgestellt, daß 48 Stunden nach Zugabe von 100 oder 200 mmol NaCl oder KCl/l zu einer handelsüblichen Fettemulsion eine signifikante Vergrößerung des Durchmessers der Tröpfchen auftritt, die auf eine Verminderung des negativen Potentials, das auf ionisierten Gruppen des Emulgators beruht und durch elektrostatische Abstoßung die Stabilität der Emulsion bewirkt, durch die einverleibten Kationen zurückzuführen ist. Bei Zusatz zweiwertiger Kationen tritt dieser Effekt in verstärktem Maße auf. Der Zusatz von 3,4 mmol $CaCl_2$/l bewirkt innerhalb von nur 4 Stunden eine Ausflockung. Die Autoren lehnen daher den Zusatz zweiwertiger Kationen zu Fettemulsionen ab und zwar auch dann, wenn gleichzeitig Aminosäuren zugesetzt werden, obwohl letztere einen gewissen stabilisierenden Effekt auf Fettemulsionen ausüben.

S.S. Davis, Adv. Clin. Nutrition - Proc. 2nd Int. Symp. Editio I.D.A., Johnson 1982, Seiten 213 - 239, weist auf die Bedeutung der abstoßenden Wirkung elektrostatischer Kräfte für die Emulsionsstabilität hin, die die anziehende Wirkung der Van der Waals-schen Kräfte überwiegen müsse. Diese elektrostatischen Kräfte sind auf eine negative Ladung der Emulgatorhülle der Fett-Tröpfchen zurückzuführen, die durch geringe Beimengungen in den handelsüblichen Phospholipiden, wie Phosphatidsäure, Phosphatidylserin und ähnliche, die als Emulgator eingesetzt werden, entsteht. Werden reine Phospholipide eingesetzt, die diese Beimengungen praktisch nicht enthalten, werden Fettemulsionen mit nur mangelnder Stabilität erhalten. Diese elektrostatischen Kräfte können durch das Zeta-Potential der Fett-Tröpfchen ausgedrückt werden, das ist das Potential, das an der Scherfläche der Teilchen auftritt, wenn sie im elektrischen Feld bewegt werden. Je größer das Zetapotential, desto stabiler ist die Emulsion.

Davis weist dabei auf die sog. "DVLO-Theorie" hin, wonach Elektrolyte das Zeta-Potential, das bei handelsüblichen Fettemulsionen bei -40 bis -50 mV liegt, reduzieren und so die beobachtete Instabilität bewirken. Bei Zusatz von NaCl wird die Stabilität der Emulsion bei überschreiten einer Konzentration von etwa 130 mmol/l zerstört. Sind verschiedenwertige Kationen zugegeben, so kumuliert deren Wirkung, wobei die destabilisierende Wirkung zweiwertiger Kationen wesentlich größer ist als jene einwertiger Kationen. Noch stärker wirken dreiwertige Kationen. Insgesamt soll gemäß DVLO-Theorie die wirksame Konzentrationen von 130 mmol/l nicht überschritten werden, wobei die Gleichung 130 mmol = a + 64 b + 779c gilt, in der a die Konzentration an einwertigen Kationen, b jene an zweiwertigen Kationen und c jene an dreiwertigen Kationen in mmol/l ist. Demnach genügen beispielsweise schon 2,1 mmol/l an zweiwertigen oder 0,18 mmol/l an dreiwertigen Kationen, um die Instabilitätsgrenze zu überschreiten. Größere als die genannten, die Stabilitätsgrenze darstellenden Konzentrationen an 2- oder 3-wertigen Kationen führen sogar zu einer Umladung der bereits instabil gewordenen Fettemulsionen zu einem positiven Zeta-Potential.

Die Arbeit von K. Sommermeyer, "Aspekte zur physikalisch-chemischen Charakterisierung, Herstellung, Kontrolle und Kompatibilitätsprüfung von Lipovenös®" in "Klinische Ernährung 21, Erfahrungen mit Fettemulsionen, Metabolische Effekte und Verträglichkeit" Seite 1 - 11, W. Zuckerschwerdt Verlag 1986, zeigt an einer handelsüblichen Fettemulsion, die mit einem Emulgator, der ein Zeta-Potential von -50 mV ergibt, hergestellt wurde, daß durch Zugabe von Natriumionen nach anfangs starker Verminderung des Zeta-potentials bei einer Konzentration von 40 mmol/l eine weitgehende Annäherung des Zeta-Potentials an den 0-Punkt erzielt wird, dieser aber auch bei weiterer Konzentrationserhöhung nicht überschritten wird. Bei Zugabe von Calciumionen hingegen genügen schon 6 mmol/l, um den 0-Punkt zu erreichen. Bei Steigerung auf bis zu 10 mmol/l wird ein positives Zeta-Potential von +4 mV eingestellt. Mit Zugabe von nahezu 1 mmol/l an Aluminium sind sogar positive Zeta-Potentiale von bis zu +40 mV erzielbar. Bei zwei- und dreiwertigen Kationen ist aber die durch die DVLO-Regel festgelegte Stabilitätsgrenze mit jenen Konzentrationen, mit denen der 0-Punkt erreicht wird, bereits überschritten.

Überraschenderweise konnte nun gefunden werden, daß im Gegensatz zur bisherigen Lehrmeinung durch Umladung von Fettemulsionen zu positiven Zeta-Potentialen eines gewissen Mindestausmaßes stabile Fettemulsionen erhalten werden, obwohl zur Erzielung dieser Umladung Konzentrationen an zwei- und/oder dreiwertigen Kationen dienen, die über der Stabilitätsgrenze gemäß DVLO-Theorie liegen. Diese positiv geladenen stabilen Fettemulsionen besitzen gegenüber den bisher bekannten stabilen Fettemulsionen mit negativer Ladung den Vorteil, daß sie mit den für die parenterale Ernährung üblichen Elektrolyten versetzt werden können, ohne dadurch an Stabilität einzubüßen. Voraussetzung für die Herstellung solcher erfindungsgemäßer positiv geladener, stabiler Fettemulsionen ist, daß für deren Herstellung Phospholipide als Emulgatoren gewählt werden, die relativ wenig, vorzugsweise kaum, negativ geladen sind.

Gegenstand der vorliegenden Erfindung ist demnach eine stabile Fettemulsion für die parenterale Verabreichung vom Typ Öl-in-Wasser mit einem Phospholipid als Emulgator, das dadurch gekennzeichnet ist, daß sie einen Gehalt an nichttoxischen zweiwertigen Metallkationen in physiologisch akzeptabler Konzentration, jedoch von maximal 30 mmol und/oder an nichttoxischen dreiwertigen Kationen in physiologisch akzeptabler Konzentration, jedoch von maximal 1 mmol/l aufweist und die disperse Phase ein positives Zetapotential von mindestens +7 mV, besitzt. Bevorzugt sind positive Zetapotentiale von 8 bis etwa +20 mV. Der Mindestgehalt an den angeführten Kationen bestimmt sich daraus, daß er ausreichen muß, um ein positives Zetapotential der dispersen Phase im geforderten Ausmaß zu erreichen. Die dazu nötigen Mengen variieren je nach eingesetztem Emulgator, wobei davon auszugehen ist, daß dreiwertige Metallkationen wie z.B. Fe(III)-Ion, wie bereits bekannt, etwa elfmal so wirksam sind, wie zweiwertige Metallkationen, wie etwa Calcium- oder Magnesiumion. Von zweiwertigen Metallkationen können bei günstiger Ausgangslage 0,5 mmol/l genügen, daß ein positives Zetapotential von +7 mV oder mehr besteht und damit eine ausreichende Stabilität erreicht ist. Vorzugsweise sollte jedoch ein Zetapotential von +8 mV bis etwa +20 mV bestehen, wozu ein Gehalt an diesen Ionen im Bereich von 1 - 10 mmol/l zu wählen ist. Die damit erzielte höhere Stabilität ist in vermehrtem Maße dann von besonderem Nutzen, wenn der Fettemulsion zur weiteren Ergänzung des Nährstoffangebots anionische Bestandteile wie z.B. saure Aminosäuren zugesetzt werden, wobei aber zu berücksichtigen ist, daß die Kurve des Anstieges des positiven Zetapotentials mit zunehmender Konzentration an Kationen verflacht, so daß Konzentrationserhöhungen über 10 mmol/l hinaus zu keiner wesentlichen weiteren Erhöhung des positiven Zetapotentials mehr führen. Ferner ist zu bedenken, daß im Zuge der Bereitung einer Nährlösung weitere Mengen, vor allem an einwertigen Kationen, jedoch auch an zweiwertigen Kationen wie etwa Zinkionen zugesetzt werden, so daß aus physiologischen Gründen und auch zur Erhaltung eines genügenden Spielraumes für die Erstellung einer Rezeptur für ein Gesamtnährsystem der Gehalt der erfindungsgemäßen Fettemulsion an zweiwertigen Kationen nicht höher als nötig gewählt werden soll. Im allgemeinen gilt jedoch, daß höhere Konzentrationen der genannten Kationen die Stabilität erhöhen, jedoch soll an zweiwertigen Kationen 30 mmol/l nicht überschritten werden, da sonst die Stabilität leidet. Bei Einsatz von dreiwertigen Kationen wie etwa dem dreiwertigen Eisenion sind die Mengen entsprechend der stärkeren Wirkung zu wählen. Es genügen meist bereits 0,05 bis 0,5 mmol/l, um ein positives Zetapotential im gewünschten Ausmaß zu erhalten. Höhere Mengen an dreiwertigem Eisenion als die als Höchstgrenze angegebenen 1 mmol/l können in manchen Fällen, vor allem dann, wenn beim Patienten kein Eisenmangel besteht, unerwünscht sein, auch wenn die Emulsion durch Zugabe weiterer Bestandteile verdünnt wird.

Für die Erzielung der positiven Ladung der erfindungsgemäßen Emulsion sind zwei- oder dreiwertige Kationen, die in Form wasserlöslicher Salze zugesetzt werden, schlechthin wirksam. Da jedoch die erfindungsgemäße Emulsion zur parenteralen Verabreichung bestimmt ist, können an Metallkationen nur solche gewählt werden, die in der zur Erzielung des erfindungsgemäßen Effektes nötigen Konzentration vom Organismus toleriert werden. Da die einzelnen Kationen unterschiedliche Toleranzgrenzen aufweisen, können keine allgemein gültigen Konzentrationsgrenzen angegeben werden. Die Angabe "in physiologisch akzeptabler Konzentration" ist demnach so zu verstehen, daß ein zur Erzielung des erfindungsgemäßen Effektes eingesetztes zwei- oder dreiwertiges Kation jeweils höchstens in einer physiologisch tragbaren Konzentration zugegen sein darf. Liegt diese so niedrig, daß damit der erfindungsgemäße Effekt noch nicht zur Gänze erreicht werden kann, ist es möglich, durch Anwendung verschiedener Kationen die positive Ladung der dispersen Phase in einem Ausmaß einzustellen, daß die erfindungsgemäße Stabilität gegeben ist. Beispielsweise könnte man eine relativ kleine Menge an Zinkion, beispielsweise 0,03 bis 0,06 mmol, einsetzen und die Restmenge an Kationen, die zur Erreichung des Zetapotentials von mindestens +7 mV oder besser mindestens +8 mV und mehr nötig ist, in Form von Calcium- und/oder Magnesiumion zugeben.

Eine besonders günstige Kombination von Kationen zur Erzielung des erfindungsgemäßen positiven Zetapotentials ist jene von dreiwertigem Eisen mit Calcium- und/oder Magnesiumion, insbesondere Calciumion, da damit mit relativ niedrigen Konzentrationen hohe positive Zetapotentiale erreicht werden können. Bei der Bemessung der Höchstgrenze der Konzentration ist dabei zu berücksichtigen, daß die Eisen(III)-Ionen 11 mal wirksamer sind als die zweiwertigen Kationen und daher von der Höchstgrenze für zweiwertige Kationen von 30 mmol/l die Eisen(III)-Konzentration multipliziert mit 11 abzuziehen ist. Eine bevorzugte Mischung ist jene von 0,02 bis 0,2 mmol/l Eisen(III)-Ion und 4 bis 9 mmol/l Calciumion.

Die tolerablen Konzentrationen an den eingesetzten Kationen sind jedem Fachmann geläufig, so daß er unschwer die entsprechenden Konzentrationen einhalten kann. Als zweiwertige Kationen sind aus physiologischen Gründen Calcium und Magnesium bevorzugt. In geringeren Mengen können auch Zink und eventuell auch zweiwertiges Kupfer Anwendung finden.

Die erfindungsgemäße Fettemulsion besitzt den besonderen Vorteil, daß sie im Gegensatz zu den bisher bekannten Fettemulsionen durch einen weiteren Elektrolytzusatz, wie er bei parenteral zu verabreichenden Lösung erwünscht und üblich ist, an Stabilität zunimmt, gleichgültig ob es sich bei den aus medizinischen Gründen zugesetzten weiteren Kationen um ein- oder mehrwertige Kationen handelt. Ist nämlich einmal die Umladung der dispersen Phase zu einer positiven Ladung vollzogen, wird durch Zusatz jeglicher weiterer Kationen die die Stabilität bewirkende positive Ladung erhöht und damit die Emulsion stabiler gemacht. Damit wächst auch die Verträglichkeit der Emulsion für andere polare Nährstoffe, insbesondere jene gegenüber basischen und auch sauren Aminosäuren und auch gegenüber Kohlenhydraten bzw. gegenüber Verdünnung, was die erfindungsgemäße Emulsion zu einer idealen Basis für die Herstellung von Gesamtnährsystemen macht.

Dieser Umstand macht es auch möglich, zur Erzielung einer besonders guten Stabilität der erfindungsgemäßen Emulsion, die später als Basis für eine Gesamtnährlösung dienen soll, zunächst eine höhere Konzentration an zwei- und/oder dreiwertigen Kationen für die Bereitung der erfindungsgemäßen Emulsion zu wählen, die in manchen Fällen sogar an der Grenze der physiologischen Verträglichkeit liegen kann. Nach Zusatz von weiteren, aus medizinischen Gründen gewünschten Elektrolyten, wie Natrium- oder Kaliumsalzen, kann dann die Emulsion durch Zusatz von Lösungen anderer Nährstoffe wie Aminosäuren oder Kohlenhydrate verdünnt und damit die Elektrolytkonzentration in den für die Verabreichung gewünschten Bereich gebracht werden, ohne daß die Stabilität darunter leidet.

Eine Voraussetzung für den Erhalt der erfindungsgemäßen, positiv geladenen, stabilen Fettemulsion ist, daß bei deren Herstellung von Phospholipiden als Emulgatoren ausgegangen wird, die relativ gering geladen sind. Während die üblicherweise für die Bereitung von Fettemulsionen verwendeten Emulgatoren, die auch handelsüblich sind, in dispergierter Form bei pH 7,4 ein negatives Zetapotential von -40 mV bis -50 mV ergeben, finden gemäß vorliegender Erfindung Phospholipide als Emulgatoren Verwendung, die in dispergierter Form bei dem genannten pH-Wert ein negatives Zetapotential von maximal -20 mV, vorzugsweise aber wesentlich weniger, nämlich von unter -5 mV, besser -2 mV ergeben, wobei Bereiche, die noch näher an 0 liegen, besonders bevorzugt sind.

Wird diese Forderung nicht eingehalten, wird lediglich die von früheren Autoren beschriebene instabile Phase erreicht, die durch die dabei beobachtete Ausflockung gekennzeichnet ist. Bei zu hoher negativer Ladung der Ausgangsemulsion vor der Umladung kann kein so hohes positives Zetapotential erreicht werden, daß der Emulsion nun durch die positive Ladung die nötigen abstoßenden elektrostatischen Kräfte verliehen werden.

Bei dreiwertigen Kationen, die an sich stärker wirken, wird diese zumeist durch die wesentlich niedrigeren Toleranzgrenzen für den menschlichen Organismus kompensiert. Viele dreiwertige Ionen wie beispielsweise das Aluminiumion scheiden wegen ihrer Toxizität überhaupt aus. Auch das unter den dreiwertigen Kationen bevorzugte dreiwertige Eisenion sollte aus physiologischen Gründen in niedrigen Konzentrationen, die meist deutlich unter der Obergrenze von 1 mmol/l liegen sollten, eingesetzt werden. Solch niedrige Konzentrationen können vorteilhaft und ohne Einbuße an Stabilität dann verwendet werden, wenn von Phospholipiden mit möglichst geringer negativer Ladung, vorzugsweise von maximal -5 mV, ausgegangen wird.

Ein weiterer Gegenstand der vorliegenden Erfindung ist demnach ein Verfahren zu Herstellung der erfindungsgemäßen, stabilen Fettemulsion, das dadurch gekennzeichnet ist, daß Öl mit Hilfe eines Phospholipids, das in wäßriger Dispersion bei pH 7,4 ein negatives Zetapotential von maximal -20 mV ergibt, in Wasser emulgiert wird, und die disperse Phase durch Zusatz von nichttoxischen zweiwertigen Metallkationen in physiologisch akzeptabler Konzentration, jedoch von maximal 30 mmol/l und/oder von nichttoxischen dreiwertigen Metallkationen in physiologisch akzeptabler Konzentration, jedoch von maximal 1 mmol/l, vor oder nach dem Emulgiervorgang auf ein positives Zetapotential umgeladen wird, wobei die Menge der zugesetzten Kationen so gewählt wird, daß ein Zetapotential von mindestens +7 mV eingestellt wird.

Es werden also die zwei- und/oder dreiwertigen Kationen entweder der wäßrigen Phase vor der Dispergierung des Fettes zugesetzt oder es werden der zunächst bereiteten Emulsion die zur Erzielung der erfindungsgemäßen Umladung nötigen Kationen zugesetzt. Vorzugsweise erfolgt im letztgenannten Fall der Zusatz unmittelbar anschließend an die Bereitung der Emulsion, vor allem dann, wenn Emulgatoren verwendet wurden, die in wäßriger Dispersion ein Zetapotential von unter -5 mV ergeben. Werden hingegen Emulgatoren im negativeren Teil des erfindungsgemäßen Bereiches eingesetzt, ist es auch möglich, den Umladungsschritt des erfindungsgemäßen Verfahrens zu einem späteren Zeitpunkt vorzunehmen.

Die Wahl der Konzentration der zugesetzten Kationen innerhalb der erfindungsgemäßen Grenzen muß die Höhe des negativen Zetapotentials des verwendeten Emulgators berücksichtigen. Ist dieses nahe an der Grenze der -20 mV, so sind höhere Ionenkonzentrationen zu wählen als in jenen Fällen, bei denen das Zetapotential des Emulgators bei -5 mV oder gar darunter liegt. Bei Verwendung zweiwertiger Kationen für den Umladungsschritt ist eine Konzentration von 0,5 mmol/l nur dann ausreichend, um das geforderte Mindestpotential von +7 mV zu erreichen, wenn der Emulgator ein negatives Zetapotential nahe 0 ergibt. Bei Einsatz eines Emulgators eines negativen Zetapotentials von -5 mV oder darunter sind 1 bis 2 mmol/l bevorzugt. Hingegen sind bei Verwendung von Phospholipiden als Emulgator, die ein negatives Zetapotential von mehr als -5 mV ergeben, höhere Konzentrationen an zweiwertigen Kationen innerhalb des erfindungsgemäßen Bereiches einzusetzen. In diesen Fällen, vor allem dann, wenn der Emulgator ein Zetapotential von -15 bis -20 mV ergibt, sind Konzentrationen an zweiwertigen Kationen von 5 bis 10 mmol/l zu bevorzugen. Bei Einsatz von Emulgatoren, die nahe der oberen Grenze des negativen Zetapotentials liegen, besteht eine bevorzugte Ausführungsform für den Umladungsschritt darin, Eisen(III)-Ion in Kombination mit zweiwertigen Kationen wie Ca- oder Mg-Ion einzusetzen. Dies erlaubt die Einstellung positiver Zetapotentiale um oder über +15 mV, die in solchen Fällen mit zweiwertigen Kationen allein kaum mehr zu erreichen sind. Besonders bevorzugt ist hierbei beispielsweise eine Kombination von 0,02 bis 0,2 mmol/l Fe(III)-Ion mit 4 bis 9 mmol/l Calciumion.

Als Phospholipide sind Phosphatidylcholine oder Phosphatidylethanolamine bevorzugt, obwohl jedes andere, im Sinne der vorliegenden Erfindung relativ gering geladene Phospholipid, wie z.B. Sphingomyelin oder Lysophospholipide, ebenfalls verwendet werden können. Die Natur des Fettsäureteiles des Phospholipids ist nicht kritisch für die erfindungsgemäße Herstellung der stabilen Fettemulsion und kann nach üblichen Kriterien, die jedem Fachmann bekannt sind, gewählt werden. Beispielsweise können Dipalmitoylphosphatidylcholin oder Dimyristoylphosphatidylcholin genannt werden. Ebenso können auch hochgereinigte Phospholipidgemische, wie sie im Handel sind, eingesetzt werden, sofern sie in Dispersion ein negatives Zetapotential von nicht höher als -20 mV ergeben, wobei hierunter das Zetapotential der Phospholipidmischung zu verstehen ist. Dazu sollte der Anteil an geladenen Phospholipiden, wie z.B. Phosphatidsäure, Phosphatidylserin, Phosphatidylinositol oder Phosphatidylglycerin 2 Gew.-% nicht überschreiten, vorteilhaft sind Anteile von 0,5 Gew.-% oder darunter, z.B. solche von 0,1 Gew.-%. Bevorzugt ist deren Anteil so niedrig wie möglich.

Als Ölkomponente können alle jene Öle, wie z.B. Sojaöl, Distelöl, Olivenöl, Maiskeimöl, etc. dienen, die für die Herstellung von Fettemulsionen zur parenteralen Verabreichung üblich sind. Ebensogut können aber auch Öle, wie Leinöl, Fischöle etc., die reich an Omega-3-Fettsäuren sind, eingesetzt werden, und schließlich kann auch ein Gehalt an mittelkettigen Triglyceriden vorgesehen werden. Üblicherweise ist der Fettgehalt der Emulsion 3 bis 30 Gew.-% vorzugsweise beträgt er 10 bis 20 Gew.-%. Die Menge an Phospholipid wird wie üblich mit etwa 1 - 2 %, bezogen auf die Emulsion, gewählt, unabhängig von der darin enthaltenen Fettmenge. Letztere beeinflußt daher auch die erfindungsgemäß zu wählende Konzentration an den zwei- und/oder dreiwertigen Kationen pro Liter der Fettemulsion nicht.

Die erfindungsgemäße Fettemulsion kann als solche parenteral verabreicht werden, bevorzugt wird sie aber für die Herstellung von Gesamtnährsystemen eingesetzt. Hierbei kann aufgrund ihrer Stabilität entweder das Gesamtnährsystem in den Handel gebracht werden, oder es können der erfindungsgemäßen Fettemulsion später die gewünschten Nährstoffe, Elektrolyte, Spurenelemente und/oder Vitamine zugesetzt werden. Die Substanzen, die der Fettemulsion gemäß vorliegender Erfindung zugesetzt werden, um ein Gesamtnährsystem herzustellen, werden nach den auf diesem Fachgebiet üblichen Kriterien gewählt, wobei auf folgende Publikationen verwiesen werden kann, die als Referenz einbezogen werden: du Plessis et al, J. Clin. Pharm. Ther. (1987) 12, 307-318; Whateley et al., J. Clin. Hosp. Pharm. (1984) 9, 113-126; Kawilarang et al., ibid. (1980), 5 151-160; und Thomas, Aust. J. Hosp. Pharm., (1987) 17 (2), 115-117.

In manchen speziellen Fällen, vor allem deshalb, um für die Wahl der Zusammensetzung der Elektrolyte die optimale Bandbreite zur Verfügung zu haben, kann es erwünscht sein, für die Bereitung des Gesamtnährsystems von der noch gering negativ geladenen Fettemulsion, die noch keine zwei- und/oder dreiwertigen Kationen enthält, auszugehen, und dieser Emulsion sowohl die zur Umladung nötigen Kationen als auch die übrigen Bestandteile zuzusetzen, wobei die Reihenfolge des Zusatzes für den Erfolg nicht ausschlaggebend ist. In einem solchen Fall muß mit gewissen Lagerzeiten der noch negativ geladenen Emulsion gerechnet werden, so daß sich für diesen Fall empfiehlt, ein Phospholipid als Emulgator einzusetzen, das in Dispersion ein negatives Zetapotential von -15 bis -20 mV ergibt, da sonst die zur Lagerung nötige Stabilität nicht gegeben wäre. Eine solche negativ geladene Emulsion kann durch Autoklavieren sterilisiert werden.

Für die Bestimmung des Zetapotentials wird die Wanderungsgeschwindigkeit durch konventionelle Partikel-Elektrophorese-Methoden in einem bekannten elektrischen Feld gemessen. Eine solche Methode wurde z.B. von Bangham A.D., Flemans R., Heard D.H. and Seaman G.V.F., 1958, in Nature 182, Seite 642, beschrieben. Bei der Messung kann vorteilhaft die Laser-Doppler-Velocimetrie eingesetzt werden. Das positive Zetapotential der erfindungsgemäßen Fettemulsion wird an dieser selbst gemessen, das negative Zetapotential der für die erfindungsgemäße Herstellung der Fettemulsion dienenden Phospholipide wird in einer Dispersion des Phospholipids in einem wäßrigen Medium bei pH 7,4 bestimmt.

Die erfindungsgemäßen Fettemulsionen sind nicht nur Stunden, sondern eine Woche und mehr, in vielen Fällen mehrere Wochen oder gar Monate stabil.

Die Erfindung wird durch die folgenden Beispiele näher erläutert

Beispiel 1

12 g eines im Handel befindlichen, zu 94% aus Phosphatidylcholin bestehenden Phospholipids, das als Dispersion in einem wäßrigen Medium bei pH 7,4 ein Zetapotential von -20 mV ergibt, und neben dem Hauptbestandteil 3 % Lysophosphatidylcholin, maximal 2 % andere Phospholipide und maximal 2 % andere Phospholipide und maximal 2 % Cholesterin enthält, wurden in 820 ml Wasser ad infundi über eine Periode von 5 Minuten in einem Silverson rotary blender dispergiert. Nach Zugabe von 200 g Sojaöl wurde weitere 10 Minuten gemischt und die so erhaltene Vormischung wurde anschließend durch einen Microfluidizer (Modell M 110, Microfluidics Corp.) in 6 Durchgängen bei 70 Mpa emulgiert. Dieser Emulsion wurden durch ein 0,2 µm-Filter 10 ml einer 0,5 molaren Calciumchloridlösung zugesetzt.

Die so erhaltene Emulsion wurde durch Autoklavieren sterilisiert. Sie enthält 20 % Fett und 5 mmol/l Calcium. Sie besitzt ein Zetapotential von +8 mV. Die durchschnittliche Partikelgröße beträgt 320 nm, gemessen durch Photon Correlation Spectroscopy.

Beispiel 2

Wie in Beispiel 1 beschrieben wurde eine Fettemulsion bereitet, mit dem Unterschied, daß dieser 20 ml einer 0,5 molaren Calciumchloridlösung zugesetzt wurden. Die so erhaltene Emulsion enthält 20 % Fett und 10 mmol/l Calcium. Sie besitzt ein Zetapotential von +10 mV.

Beispiel 3

0,12 g Dimyristoylphosphatidylcholin, das in dispergiertem Zustand in wäßrigem Medium bei pH 7,4 ein Zetapotential von -4 mV ergibt, wurde mittels Ultraschall in 8 ml Wasser dispergiert. Dieser Dispersion wurden 2 ml Soja-Öl (entsprechend der Britischen Pharmakopoe) zugesetzt. Die entstandene Mischung wurde in zwei 5-Minuten-Perioden mit Ultraschall behandelt. Zwischen den Ultraschallbehandlungen wurde eine 10 Minuten dauernde Abkühlungsperiode dazwischengeschaltet, während der durch Eintauchen in Eis gekühlt wird. Der so erhaltenen Emulsion wurden 0,01 mmol Calciumchlorid in wäßriger Lösung, entsprechend einem Calciumion-Gehalt von 1 mmol/l zugesetzt. Anschließend wurde die Emulsion durch Autoklavieren sterilisiert. Sie besitzt ein Zeta-Potential von +12 mV.

Beispiel 4

Einer gemäß Beispiel 2 bereiteten Emulsion wurden zu Umladung 0,02 mmol Calciumchlorid, entsprechend einem Ca-Ionen Gehalt von 2 mmol/l zugesetzt. Sie besitzt ein Zeta-Potential von +18 mV.

Beispiel 5

Werden der nach Beispiel 2 bereiteten, negativ geladenen Emulsion zur Umladung 0,04 mmol Calciumchlorid zur Einstellung eines $Ca^{++}$-Gehaltes von 4 mmol/l zugefügt, besitzt die Emulsion ein Zetapotential von +23 mV.

Beispiel 6

Werden der gemäß Beispiel 2 bereiteten negativ geladenen Emulsion zur Umladung 0,1 mmol Calciumchlorid zur Einstellung eines $Ca^{++}$-Gehaltes von 10 mmol/l zugefügt, besitzt die so erhaltene Emulsion ein Zetapotential von +30 mV.

Die Emulsion gemäß den Beispielen 3 - 6 zeigen alle eine gute Stabilität, gemessen am Maß der Ausflockung, das auf turbidimetrischem Wege über die Höhe der Lichtabsorption nach Verdünnung auf 0,1 % Ölphase bestimmt wird.

Beispiel 7

Wie im Beispiel 1 beschrieben, wurde eine Fettemulsion bereitet mit dem Unterschied, daß dieser 20 ml einer 0,5 molaren Calciumchloridlösung und 10 ml einer 0,1 molaren Eisen(III)chloridlösung zugesetzt wurde. Die so erhaltene Emulsion enthält 20 % Fett, 10 mmol/l Calcium und 1 mmol/l Eisen. Sie ergibt ein Zetapotential von +64 mV.

Beispiel 8

Gemäß Beispiel 1, jedoch ohne Zusatz von Calciumchlorid, wurde eine Emulsion bereitet, die ein Zetapotential von -12 mV besitzt. Diese Emulsion wurde durch Autoklavieren sterilisiert.

Zu 250 ml dieser Emulsion wurden zwecks Bereitung von 1,1 l eines Gesamtnährsystems folgende Nährstofflösungen in sterilisierter Form zugegeben:

1. 250 ml einer 60 %igen Glucoselösung,
2. 100 ml eines Elektrolytkonzentrates enthaltend

| | |
|---|---|
| Na | 80,0 mmol |
| K | 40,0 mmol |
| Cu | 1,2 mmol |
| Mg | 7,0 mmol |
| Zn | 0,046 mmol |
| Mn | 0,005 mmol |
| Fe-III | 0,018 mmol |
| Chlorid | 14,1 mmol |
| Malat | 40,0 mmol |
| Phosphat | 40,0 mmol |

3. 500 ml einer Aminosäurenlösung enthaltend:

| | |
|---|---|
| L-Isoleucin | 1,750 g |
| L-Leucin | 3,150 g |
| L-Lysin | 3,250 g |
| L-Methionin | 0,950 g |
| L-Phenylalanin | 2,250 g |
| L-Threonin | 2,100 g |
| L-Tryptophan | 0,750 g |
| L-Valin | 3,100 g |
| L-Arginin | 4,000 g |
| L-Histidin | 3,000 g |
| L-Alanin | 7,750 g |
| L-Asparaginsäure | 1,050 g |
| L-Cystein | 0,525 g |
| L-Glutaminsäure | 4,653 g |
| Glycin | 3,150 g |
| L-Ornithin | 1,251 g |
| L-Prolin | 4,000 g |
| L-Serin | 2,350 g |
| L-Tyrosin | 0,750 g |

Es wurden also insgesamt 7,051 mmol zweiwertige Kationen und 0,018 mmol dreiwertiges Eisen zugesetzt, das entspricht einem Gehalt von 6,41 mmol/l an zweiwertigen Kationen und 0,016 mmol/l $Fe^{+++}$.

7

In dem resultierenden Gesamtnährsystem konnte unter Verwendung von Laser-Diffraction als größter bestimmbarer Partikel (Nachweisgrenze 0,1 %) ein solcher von 4 μm festgestellt werden. Das System entsprach also der Eur. Ph. Während einer Lagerzeit von 30 Tagen bei +4°C war keine Veränderung des größten bestimmbaren Partikels feststellbar.

Die gleichen Ergebnisse wurden erhalten, wenn dem Gesamtnährsystem zusätzlich noch Calciumionen in einer Menge zugegeben wurden, die 10 mmol Ca$^{++}$/l entspricht. Die Konzentration an zweiwertigen Kationen wurden damit auf 16,4 mmol/l erhöht.

Wurden 250 ml einer handelsüblichen 20 %igen Fettemulsion mit einem negativen Zetapotential von -45 mV mit den gleichen Lösungen 1., 2. und 3. versehen, so betrug der größte bestimmbare Partikel unmittelbar nach der Mischung ebenfalls 4 μm. Der gleiche Befund wurde erhoben, wenn auch bei dieser Mischung durch Zusatz von Calciumionen der Gehalt an zweiwertigen Kationen auf 16,4 mmol/l erhöht wurde.

Diese beiden auf einer handelsüblichen Fettemulsion basierenden Gesamtnährsysteme wurden ebenso wie das erfindungsgemäß hergestellte Gesamtnährsystem einer Lagerung bei +4°C unterworfen. Nach 25 Tagen Lagerzeit stieg der größte bestimmbare Partikel im System ohne Calciumzusatz (also mit einem Gehalt an 6,4 mmol/l an zweiwertigen Kationen) auf 15 μm, jener bei Zusatz von 10 mmol/l Ca. auf 12 μm. Nach 30 Tagen waren sowohl das System ohne Calciumzusatz als auch jenes mit Calciumzusatz so zersetzt, daß eine Reihe von Partikeln außerhalb der Bestimmungsgrenze des verwendeten Gerätes lagen.

## Patentansprüche

1. Stabile Fettemulsion für die parenterale Verabreichung vom Typ Öl-in-Wasser mit einem Phospholipid als Emulgator, dadurch gekennzeichnet, daß sie einen Gehalt an nichttoxischen zweiwertigen Metallkationen in physiologisch akzeptabler Konzentration, jedoch von maximal 30 mmol/l und/oder an nichttoxischen dreiwertigen Metallkationen in physiologisch akzeptabler Konzentration, jedoch von maximal 1 mmol/l aufweist, und die disperse Phase ein positives Zetapotential von mindestens +7 mV, bestimmt durch Messung der Wanderungsgeschwindigkeit mittels Konventioneller Partikel-Elektrophorese - Methoden in einem bekannten Elektrischen Feld besitzt.

2. Fettemulsion nach Anspruch 1, dadurch gekennzeichnet, daß das Zetapotential der dispersen Phase +8 bis +20 mV beträgt.

3. Fettemulsion nach Anspruch 1, dadurch gekennzeichnet, daß sie zweiwertige Kationen in einer Konzentration von mindestens 0,5 mmol/l enthält.

4. Fettemulsion nach Anspruch 3, dadurch gekennzeichnet, daß die Konzentration 1 - 10 mmol/l beträgt.

5. Fettemulsion nach den Ansprüchen 1 bis 4, dadurch gekennzeichnet, daß das zweiwertige Kation Calcium- und/oder Magnesiumion ist.

6. Fettemulsion nach den Ansprüchen 1 und 2, dadurch gekennzeichnet, daß sie einen Gehalt an 0,02 bis 0,2 mmol/l an dreiwertigem Eisenion und einen Gehalt an 4 bis 9 mmol/l an Calciumion aufweist.

7. Fettemulsion nach den Ansprüchen 1 bis 6, dadurch gekennzeichnet, daß sie außerdem für die parenterale Ernährung übliche, andere Nährstoffe, Elektrolyte, Spurenelemente und/oder Vitamine enthält.

8. Verfahren zur Herstellung einer stabilen Fettemulsion gemäß den Ansprüchen 1 bis 7, dadurch gekennzeichnet, daß Öl mit Hilfe eines Phospholipids, das in wäßriger Dispersion bei pH 7,4 ein negatives Zetapotential von maximal -20 mV ergibt, in Wasser emulgiert wird, und die disperse Phase durch Zusatz von nichttoxischen zweiwertigen Metallkationen in physiologisch akzeptabler Konzentration, jedoch von maximal 30 mmol/l und/oder von nichttoxischen dreiwertigen Metallkationen in physiologisch akzeptabler Konzentration, jedoch von maximal 1 mmol/l, vor oder nach dem Emulgiervorgang auf ein positives Zetapotential umgeladen wird, wobei die Menge der zugesetzten Kationen so gewählt wird, daß ein Zetapotential von mindestens +7 mV eingestellt wird.

9. Verfahren nach Anspruch 8, dadurch gekennzeichnet, daß ein Phospholipid mit einem Zetapotential von maximal -5 mV eingesetzt wird und die Umladung durch Zugabe zweiwertiger Metallkationen in einer Konzentration von 1 - 2 mmol/l vorgenommen wird.

10. Verfahren nach Anspruch 8, dadurch gekennzeichnet, daß ein Phospholipid mit einem Zetapotential von -15 bis -20 mV eingesetzt wird, und die Umladung durch Zugabe von zweiwertigen Metallkationen in einer Konzentration von 5 - 10 mmol/l oder von einem Gemisch von 0,02 bis 0,2 mmol/l an dreiwertigem Eisenion mit 4 bis 9 mmol/l an Calciumion erfolgt.

11. Verfahren nach den Ansprüchen 8 bis 10, dadurch gekennzeichnet, daß als zweiwertiges Kation Ca- und/ oder Mg-Ion eingesetzt wird.

12. Fettemulsion vom Typ Öl-in-Wasser für die Herstellung einer Fettemulsion gemäß Anspruch 1, dadurch gekennzeichnet, daß sie als Emulgator ein Phospholipid mit einem Zetapotential von -15 bis -20 mV enthält.

13. Fettemulsion gemäß den Ansprüchen 1 bis 7 zur Anwendung für die Herstellung eines insbesondere elektrolythaltigen Gesamtnährsystems.

## Claims

1. A stable fat emulsion for parenteral administration of the oil-in-water type with a phospholipid emulsifier, which contains non-toxic doubly charged metal cations in physiologically acceptable concentration but not exceeding 30 mmol/l and/or contains non-toxic triply charged metal cations in physiologically acceptable concentration but not exceeding 1 mmol/l, and wherein the disperse phase has a positive zeta potential of at least +7 mV, determined by measurement of the migration rate by means of conventional particle electrophoresis methods in a known electric field.

2. A fat emulsion as claimed in claim 1, wherein the zeta potential of the disperse phase is from +8 to +20 mV.

3. A fat emulsion as claimed in claim 1, which contains doubly charged cations in a concentration of at least 0.5 mmol/l.

4. A fat emulsion as claimed in claim 3, wherein the concentration is 1 - 10 mmol/l.

5. A fat emulsion as claimed in claims 1 to 4, wherein the doubly charged cation is calcium and/or magnesium ion.

6. A fat emulsion as claimed in claims 1 and 2, which contains from 0.02 to 0.2 mmol/l of triply charged iron ion and contains from 4 to 9 mmol/l of calcium ion.

7. A fat emulsion as claimed in claim 1 to 6, which additionally contains other nutrients, electrolytes, trace elements and/or vitamins customary for parenteral nutrition.

8. A process for preparing a stable fat emulsion as claimed in claim 1 to 7, which comprises emulsifying oil in water with the aid of a phospholipid which gives a negative zeta potential not exceeding -20 mV in aqueous dispersion at pH 7.4, and changing the charge on the disperse phase by adding non-toxic doubly charged metal cations in physiologically acceptable concentration but not exceeding 30 mmol/l and/or adding non-toxic triply charged metal cations in physiologically acceptable concentration but not exceeding 1 mmol/l, before or after the emulsification process, to a positive zeta potential, the amount of added cations being chosen so that a zeta potential of at least +7 mV is set up.

9. A process as claimed in claim 8, wherein a phospholipid with a zeta potential not exceeding -5 mV is employed, and the charge is changed by adding doubly charged metal cations in a concentration of 1 - 2 mmol/l.

10. A process as claimed in claim 8, wherein a phospholipid with a zeta potential of from -15 to -20 mV is employed, and the charge is changed by adding doubly charged metal cations in a concentration of 5 - 10 mmol/l or a mixture of 0.02 to 0.2 mmol/l of triply charged iron ion with from 4 to 9 mmol/l of calcium ion.

11. A process as claimed in claim 8 to 10, wherein Ca and/or Mg ion is employed as doubly charged cation.

12. A fat emulsion of the oil-in-water type for preparing a fat emulsion as claimed in claim 1, which contains as emulsifier a phospholipid with a zeta potential of from -15 to -20 mV.

**13.** A fat emulsion as claimed in claim 1 to 7 for use for preparing in particular an electrolyte-containing complete nutritional system.

## Revendications

**1.** Emulsion grasse, stable, pour l'administration parentérale, du type huile-dans-eau, avec un phospholipide comme émulsifiant, caractérisé par le fait qu'elle présente une teneur en cations métalliques bivalents, non toxiques, en concentration physiologiquement acceptable, mais de 30 mmoles/1 au maximum et/ou en cations métalliques trivalents, non toxiques, en concentration physiologiquement acceptable, mais de 1 mmole/1 au maximum et la phase dispersée présente un potentiel zéta d'au moins +7 mV, déterminé en mesurant la vitesse de migration à l'aide de méthodes classiques de l'électrophorèse de particules dans un champ électrique connu.

**2.** Emulsion grasse selon la revendication 1, caractérisée par le fait que le potentiel zéta de la phase dispersée est compris entre +8 et +20 mV.

**3.** Emulsion grasse selon la revendication 1, caractérisée par le fait qu'elle contient des cations bivalents en une concentration d'au moins 0,5 mmole/l.

**4.** Emulsion grasse selon la revendication 3, caractérisée par le fait que la concentration varie entre 1 et 10 mmoles.

**5.** Emulsion grasse selon les revendications 1 à 4, caractérisée par le fait que le cation bivalent est l'ion calcium et/ou l'ion magnésium.

**6.** Emulsion grasse selon les revendications 1 et 2, caractérisée par le fait qu'elle présente une teneur de 0,02 à 0,2 mmole/l en ion fer trivalent et de 4 à 9 mmoles/l en ion calcium.

**7.** Emulsion grasse selon les revendications 1 à 6, caractérisée par le fait qu'elle contient, en outre, d'autres substances nutritives, électrolytes, oligo-éléments et/ou vitamines usuels pour l'alimentation parentérale.

**8.** Procédé de préparation d'une émulsion grasse, stable, selon les revendications 1 à 7, caractérisé par le fait que l'on émulsifie de l'huile dans de l'eau à l'aide d'un phospholipide qui, en dispersion aqueuse, à pH 7,4, donne un potentiel zéta négatif de -20 mV au maximum, et on inverse la charge de la phase dispersée, avant ou après l'émulsification, à un potentiel zéta positif par addition de cations métalliques bivalents, non toxiques, en concentration acceptable physiologiquement, mais de 30 mmoles/l au maximum, et/ou de cations métalliques trivalents, non toxiques, en concentration acceptable physiologiquement, mais au maximum de 1 mmole/l, la quantité des cations ajoutés étant choisie de manière à établir un potentiel zéta d'au moins +7 mV.

**9.** Procédé selon la revendication 8, caractérisé par le fait que l'on utilise un phospholipide d'un potentiel zéta de -5 mV au maximum et on inverse la charge par addition de cations métalliques bivalents, en une concentration de 1 à 2 mmoles/l.

**10.** Procédé selon les revendications 8, caractérisé par le fait que l'on utilise un phospholipide d'un potentiel zéta de -15 à -20 mV et on effectue l'inversion par addition de cations métalliques bivalents, en une concentration de 5 à 10 mmoles/l, ou d'un mélange de 0,02 à 0,2 mmole/l d'ion fer trivalent et 4 à 9 mmoles/l d'ion calcium.

**11.** Procédé selon les revendications 8 à 10, caractérisé par le fait que l'on utilise, comme cation bivalent, ilion calcium et/ou l'ion magnésium.

**12.** Emulsion grasse du type huile-dans-eau pour la préparation d'une émulsion grasse selon la revendication 1, caractérisée par le fait qu'elle contient, comme émulsifiant, un phospholipide d'un potentiel zéta de -15 à -20 mV.

**13.** Emulsion grasse selon les revendications 1 à 7, utilisée pour la préparation d'un système d'alimentation totale contenant en particulier des électrolytes.